# EUROPEAN PATENT SPECIFICATION

(11) **EP 1 842 490 B1**
(45) Date of publication and mention of the grant of the patent: **14.09.2011**
(21) Application number: 06007431.7
(22) Date of filing: 07.04.2006
(51) Int. Cl.: A61B 17/00

(54) **Occlusion devices for treating of congenital heart disease with auto-adjusting function**
Selbstanpassende Verschlussvorrichtung zur Behandlung einer angeborenen Herzkrankheit
Dispositif d'occlusion à auto-ajustement pour le traitement d'une cardiopathie congénitale

(43) Date of publication of application: 10.10.2007
(73) Proprietor: Lifetech Scientific (Shenzhen) Co., Ltd., Shenzhen (CN)
(72) Inventor: Zang, Shixian, Shenzhen (CN); Zi, Eric, Shenzhen (CN); Lv, Shiwen, Shenzhen (CN); Feng, Yaoting, Shenzhen (CN); Xie, Yuehui, Shenzhen (CN); Zhang, Zhiwei, Shenzhen (CN); Kong, Xiangqing, Shenzhen (CN)
(74) Representative: Prinz & Partner

(56) References cited:
- EP-A- 1 576 929
- EP-A- 1 602 332
- WO-A-2005/034738
- US-A- 5 702 421
- US-A- 5 944 738
- US-A1- 2004 073 242
- US-A1- 2005 065 547

## Description

The present invention relates to an occlusion device for treating congenital heart disease, such as patent foramen ovale (PFO), atrial septal defect (ASD), patent ductus arteriosus (PDA) or ventricular septal defect (VSD).

Congenital heart diseases include patent foramen ovale (PFO), atrial septal defect (ASD), patent ductus arteriosus (PDA) and ventricular septal defect (VSD). PFO and ASD are openings in the wall between the right atrium and left atrium of the heart, which create the possibility that the blood could pass from the right atrium to the left atrium. The defect size of PFO is usually smaller than that of ASD and the left atrial defect is not concentric with that of the right atrium. Once the occluder is in place, it will effectively prevent thrombus entering into the left atrium. The atrial septal defect (ASD) is usually bigger. Currently there are many types of endocardiac occlusion devices for treating congenital heart disease, and these occluders are delivered to the desired location by a corresponding catheter.

Mechanical occlusion devices for treating congenital heart disease have been proposed in the past, some of which are disclosed in Franker et al., Chinese patent no. 1218379 ; Franker et al., Chinese patent no. 1269707; and Michael et al., Chinese patent no. 1283973. This kind of device includes a collapsible support mesh and a biocompatible membrane and the membrane is connected to the circumference of the support mesh. The support mesh, which is first put into the catheter, is delivered to the desired location, and then deployed to close the defect. This kind of device is easy to withdraw and has excellent self-centering properties.

However, the left disc of this device is in direct contact with blood, which can lead to thrombus and to the release harmful metal elements. Moreover, because the two discs are composed as a whole, the device cannot automatically adjust the angle to the unique anatomy of the patient. Meanwhile it has been found that the left disc does not deploy completely after being released. In addition, it is very difficult to precisely determine the size and shape of the defect with the existing techniques. The limitations on waist size will bring many difficulties to physicians, such as selection error. If a larger size is selected, the occluder will form a cucurbit shape and the closing effect is not perfect.

The U.S. patent application 2005/65547 discloses an ASD closure device with a self-centering effect through the use of an arm network. The left and right discs of the device are formed of a series of arms that provide tension to hold the left and right sides in place. The ends of the arms are connected to a hoop, which is covered by a sheet material. The respective left and right side arms are fixed to a center post.

Accordingly, it would be advantageous to provide a reliable device which can automatically adjust the angle to adapt to the unique anatomy of the patient.

### Summary of the invention

The present invention as defined in claim 1 provides a reliable occlusion device with an auto-adjusting function which can adapt to the unique anatomy of the patient. The two discs can attach to the defect closely, so as to increase the closing ability. Thrombus formation can be reduced because the left disc is covered with a membrane.

The right disc is made from a double-deck collapsible wire mesh and the left disc is made from at least two skeleton wires which form a plane. The plane is covered with a membrane. The two discs are linked together by the skeleton wires being passed through the mesh of the right disc.

The middle segment of each skeleton wire is U-shaped, and the depths of the troughs of the U shape are different, so the skeleton wires form a plane after being linked together. They are then covered with membranes to form a disc shape.

The left disc is produced from several radial-extending skeleton wires. After overlapping the middle segments, each skeleton wire extends radially from the center. The two ends of each skeleton wire have a spherical shape, which is wrapped by the membrane. The ends of the right disc are fixed by a tip and a joint, and the right disc undergoes heat treatment. The skeleton wires are passed through the mesh near the tip and are overlapped with one another. The membranes are made from biocompatible materials.

Furthermore, the connection between the two discs has a gimbal-like function and the distances between the two discs may expand and contract suitably, so that the device can better adapt to the unique anatomy of the patient. Therefore the two discs may attach to the defects closely and increase its closing ability. Furthermore, the device can reduce thrombus formation as well as the release of harmful metal elements because the left disc is covered with membranes. In addition, the device has a split structure, i.e. its two discs deploy completely. This makes it easy to operate and increases the closing reliability.

### Brief description of the drawings

FIG. 1 is a schematic representation of a disc which is constructed by skeleton wires according to an embodiment.
FIG. 2 is a side view of the right disc.
FIG. 3 is a side view of the skeleton wire.
FIG. 4 is a side view of the occlusion.
FIG. 5 is an enlarged partial view of a section shown in FIG. 4.
FIG. 6 is a front view of the occlusion device.
FIG. 7 and FIG. 8 are a side views of other alternative skeleton wires.
FIG. 9 is a side view of the membrane used to cover the skeleton wires.
FIG. 10 is a side view of the skeleton wires, which have been covered with the membrane as shown in FIG. 9.
FIG. 11 is a schematic representation of a mould, which is used to heat-treat the skeleton wires.
FIG. 12 is a schematic representation of a PFO occluder being released from a delivery catheter.
FIG. 13 is a schematic representation of an ASD occluder being released from a delivery catheter.
FIG. 14 is a side view of a PDA occlusion device.

### Detailed description of embodiments

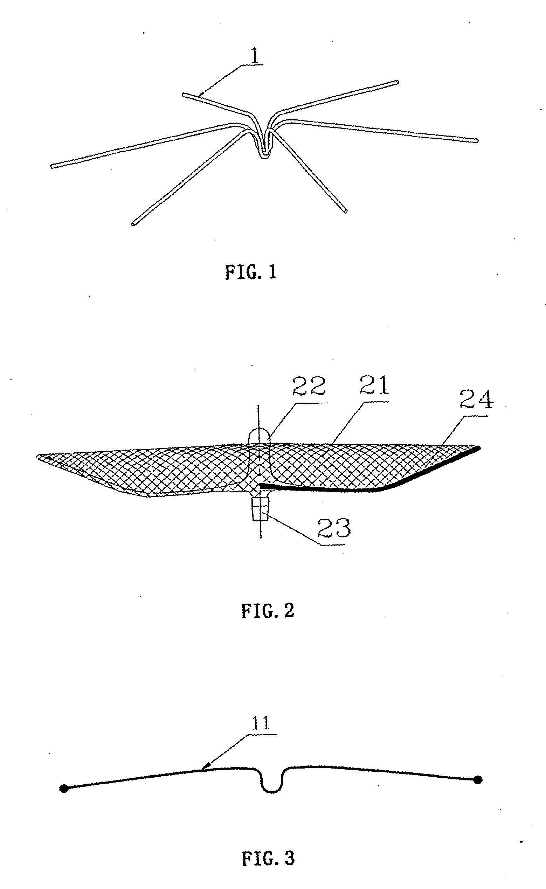

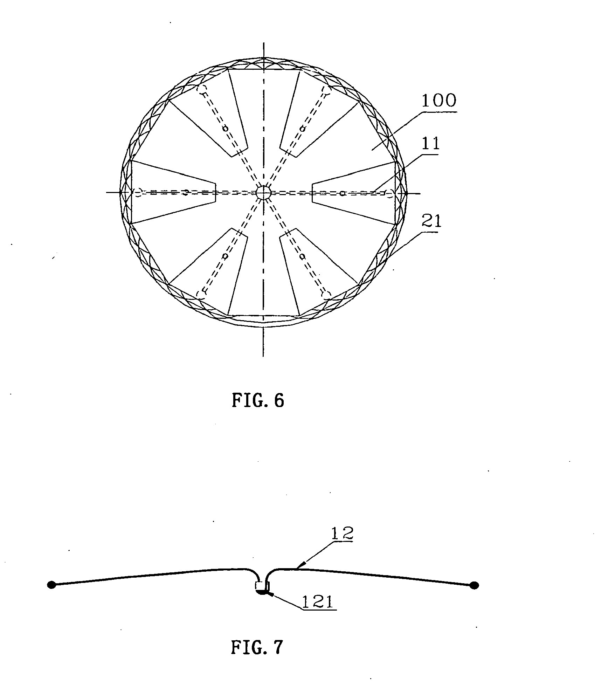

An occlusion device is provided for occluding an anatomical aperture, such as a patent foramen ovale occluder as shown in Figs. 1, 2 and 4. The occluder comprises right disc 21 (i.e. metal mesh disc), tip 22, joint 23 and a left disc 1. The left disc 1 is covered with a membrane 100 as shown in Figs. 6, 9 and 10.

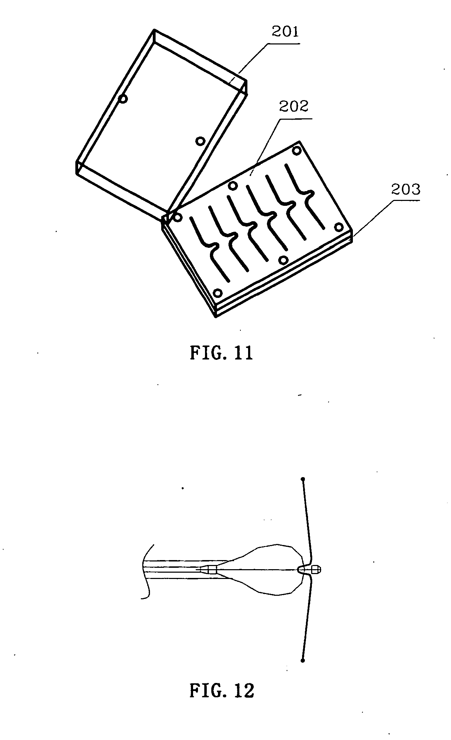

A PFO occluder will be selected as an example. Compared to the devices in the above cited patents, the left disc 1 comprises six skeleton wires 11 which are evenly distributed. The six skeleton wires are linked together at the center and form a radial-extending disc as shown in Fig. 1. The left disc 1 may comprise at least two skeleton wires 11 and the wires 11 are made from nitinol wire with shape memory. Fig. 11 illustrates the mould which is used to heat treat the skeleton wires11. The mould includes upper-mould 201, middle-mould 202 and under-mould 203 and the nitinol wire is put into the rabbet of the middle-mould 201. By heating the nitinol wire above a certain phase transition temperature, the crystal structure of the nitinol wire can be set, when in its austenitic phase. This then 'sets' the shape of the device, i.e. it keeps the shape of the mould and can keep the "set "shape invariable even if cooled.

The middle segment of each skeleton wire 11 is U-shaped and the depth of the trough of each U shape is different. Due to these different depths, these skeleton wires form a plane after being overlapped together. They are then covered with a membrane to form the left disc 1.

The right disc 21 of the PFO occluder uses moulded components. Firstly, the suitable tubular metal mesh is formed by weaving or laser carving and then the tubular metal mesh is inserted into a mould and undergoes a heat treatment. The tip 22 and joint 23 are welded to the disc as shown in Fig. 2.

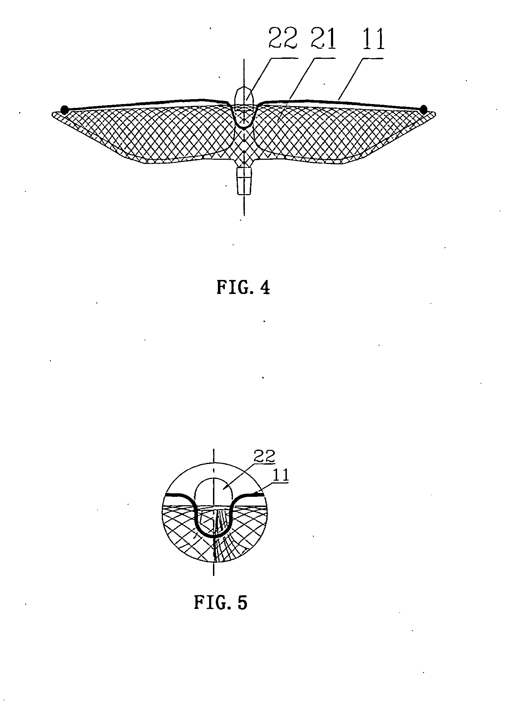

The two ends of of the skeleton wires 11 are welded into spheres, respectively, as shown in Fig. 3. The wires 11 are passed through the right disc 21 near the tip 22 to form a double-disc structure as shown in Fig. 5. As shown in Fig. 6, the skeleton wires 11 are distributed evenly and as shown in Fig. 4 a metal disc is formed.

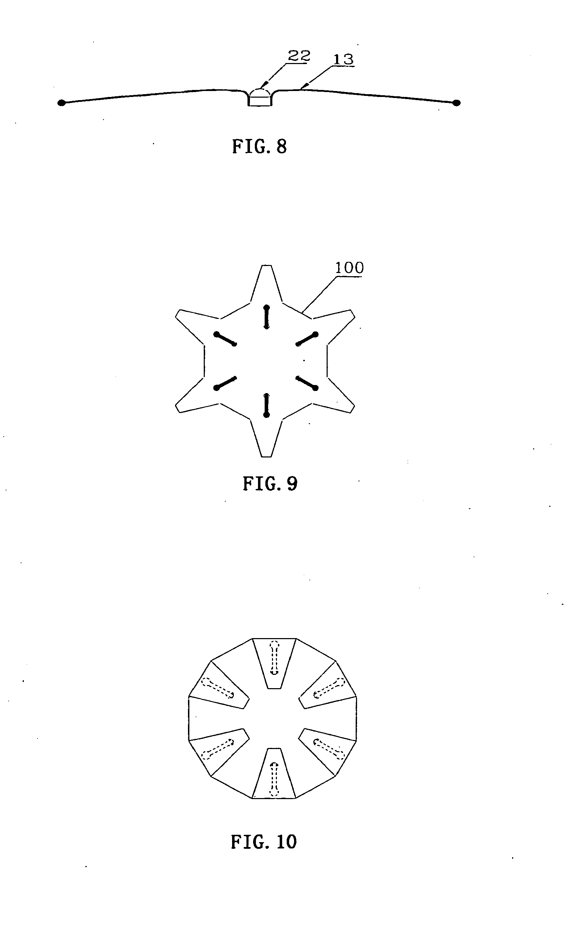

The two sides of the left disc 1 are covered with membranes as shown in Figs. 9 and 10, where the skeleton wires 11 are covered by the membrane 100. The membranes are made from biocompatible materials. The spherical ends of each skeleton wire are wrapped in the biocompatible material, so as to prevent the wire 11 from puncturing the membrane 100. Another membrane made by biocompatible material 24 is filled into the right disc 21 as shown in Fig. 2.

As described above, the left disc 1 passes through right disc 21 at the overlapping point of the wires of the left disc and the connection between the two discs has a gimbal function. Furthermore, the two discs tend to shrink or contract inwardly. The occlusion device may swing randomly, i.e., the left disc 1 and the right disc 21 may be parallel or have an angle. Accordingly, the device can adapt to the unique anatomy of the patient and the two discs can attach closely to the defect. Alternative structures of the skeleton wire 11 are shown in Figs. 7 and 8.

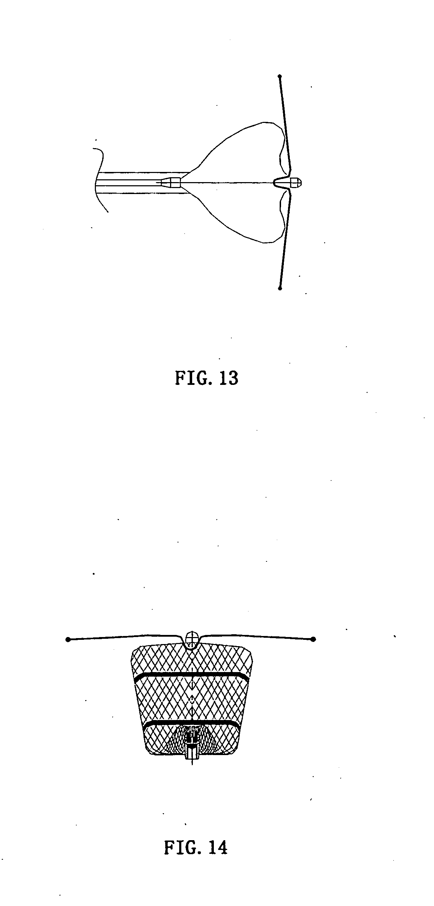

The occlusion device as described above may be extended and put into a catheter. The device is delivered to the desired location and then is released ( see Figs. 12 and 13). The tapered waist of the device not only ensures its self-centering, but can also reduce the probability of a bad occlusion result due to a size selection error. The left disc, which comprises skeleton wires and membranes, decreases the metal surface area, which reduces thrombus formation as well as the release of harmful metal elements. The two discs are individual components and can deloy completely after being released. This can avoid formation of a cucurbit shape and will increase the reliability of occlusion.

Figs 12 and 13 illustrate the deployment process of a PFO occluder and an ASD occluder, respectively. The occluder has excellent self-centering properties due to the closeness of the right disc 21 to the left disc 1.

The present invention is also suitable for the treatment of PDA and VSD defects. The only difference compared with the above occluders is that the metal mesh of a PDA occluder will not form a disc, but a "waist" as shown in Fig. 14.

The above description should not be construed as limiting the scope of the invention but as merely providing illustrations of some of the presently preferred embodiments of this invention.

## Claims

1. An occlusion device for treating congenital heart disease having an auto-adjusting function for adapting to the unique anatomy of the patient, said device comprising a collapsible right disc (21) and a left disc (1) made from at least two skeleton wires (11), said skeleton wires (11) having a radially extending portion and a U-shaped middle segment, wherein the depth of the U-shaped segment is different for each skeleton wire (11), such that the radially extending portions of the skeleton wires (11) form a plane, said plane being covered with a membrane,
**characterized in that**
said right disc (21) is made of a metal mesh and said skeleton wires (11) of said left disc (1) form said plane after being linked together with one another, and
said skeleton wires (11) pass through the metal mesh of said right disc (21) to link the right disc (21) with the left disc (1), such that the discs (1, 21) of the occlusion device may swing randomly, so as to be parallel or have an angle with respect to one another.

2. The device according to claim 1, wherein said left disc (1) is made from several radially extending skeleton wires (11) and is covered with membranes (100) on both sides of the plane for stopping blood flow.

3. The device according to claim 2, wherein the two ends of said skeleton wires (11) are of spherical shape and the two spheres are wrapped in a membrane (100) on both sides of the plane.

4. The device according to any one of the claims 1 to 3, wherein the right disc (21) is formed from a tubular metal mesh, which undergoes heat treatment to form a disc structure and which is fixed at either end by a tip (22) and a joint (23), respectively.

5. The device according to claim 4, wherein said skeleton wires (11) pass through the metal mesh of the right disc (21) near the tip (22).

6. The device according to claim 5, wherein said skeleton wires (11) overlap each other, as seen in axial direction of the left disc (1), when passed through the metal mesh near the tip (22) of the right disc (21).

7. The device according to any one of the claims 1 to 6, wherein said membranes (100) are made of biocompatible materials.

8. The device according to claim 1, wherein said skeleton wires (11) are made from nitinol wire with shape memory.

9. The device according to claim 1, wherein said left and right discs (1, 21) tend to contract inwardly toward one another and may be oriented parallel to one another or have an angle, so as to provide said auto-adjusting function for defects with different thickness and to allow a close attachment to the defect.

## Patentansprüche

1. Verschlussvorrichtung zur Behandlung einer angeborenen Herzkrankheit, die eine Selbsteinstellungsfunktion zur Anpassung an die spezielle Anatomie des Patienten aufweist, wobei die Vorrichtung eine zusammenfaltbare rechte Scheibe (21) und eine linke Scheibe (1) aus wenigstens zwei Gerüstdrähten (11) umfasst, wobei die Gerüstdrähte (11) einen sich radial erstreckenden Abschnitt und ein U-förmiges Mittelsegment aufweisen, wobei die Tiefe des U-förmigen Segments bei jedem Gerüstdraht (11) so unterschiedlich ist, dass die sich radial erstreckenden Abschnitte der Gerüstdrähte (11) eine Ebene bilden, wobei die Ebene mit einer Membran abgedeckt ist,
**dadurch gekennzeichnet, dass**
die rechte Scheibe (21) aus einem Metallgeflecht hergestellt ist und die Gerüstdrähte (11) der linken Scheibe (1) die Ebene bilden, nachdem sie miteinander verbunden worden sind, und
die Gerüstdrähte (11) das Metallgeflecht der rechten Scheibe (21) durchtreten, um die rechte Scheibe (21) mit der linken Scheibe (1) so zu verbinden, dass die Scheiben (1, 21) der Verschlussvorrichtung beliebig schwenkbar sind, so dass sie zueinander parallel sind oder einen Winkel zueinander aufweisen.

2. Vorrichtung nach Anspruch 1, bei der die linke Scheibe (1) aus mehreren sich radial erstreckenden Gerüstdrähten (11) hergestellt ist und auf beiden Seiten der Ebene mit Membranen (100) abgedeckt ist, um den Blutfluss anzuhalten.

3. Vorrichtung nach Anspruch 2, bei der die beiden Enden der Gerüstdrähte (11) eine Kugelform aufweisen und die beiden Kugeln auf beiden Seiten der Ebene in eine Membran (100) gewickelt sind.

4. Vorrichtung nach einem der Ansprüche 1 bis 3, bei der die rechte Scheibe (21) aus einem rohrförmigen Metallgeflecht gebildet ist, das zur Bildung einer Scheibenstruktur einer Wärmebehandlung unterzogen wird und das an beiden Enden durch eine Spitze (22) bzw. ein Gelenk (23) befestigt ist.

5. Vorrichtung nach Anspruch 4, bei der die Gerüstdrähte (11) das Metallgeflecht der rechten Scheibe (21) nahe der Spitze (22) durchtreten.

6. Vorrichtung nach Anspruch 5, bei der die Gerüstdrähte (11), in axialer Richtung der linken Scheibe (1) gesehen, einander überlappen, wenn sie nahe der Spitze (22) der rechten Scheibe (21) durch das Metallgeflecht geführt werden.

7. Vorrichtung nach einem der Ansprüche 1 bis 6, bei der die Membranen (100) aus biokompatiblen Materialien bestehen.

8. Vorrichtung nach Anspruch 1, bei der die Gerüstdrähte (11) aus Nitinoldraht mit Formgedächtnis hergestellt sind.

9. Vorrichtung nach Anspruch 1, bei der die linke und die rechte Scheibe (1, 21) dazu neigen, sich nach innen zueinander hin zusammenzuziehen, und parallel zueinander ausgerichtet werden können oder einen Winkel aufweisen können, um die Selbsteinstellungsfunktion für Fehler unterschiedlicher Dicken bereitzustellen und eine enge Befestigung an dem Fehler zu ermöglichen.

## Revendications

1. Dispositif d'occlusion pour le traitement de cardiopathie congénitale, comportant une fonction d'auto-ajustement pour l'adaptation à l'anatomie propre au patient, le dispositif comprenant un disque droit (21) repliable et un disque gauche (1) constitué par au moins deux fils métalliques d'ossature (11), les fils métalliques d'ossature (11) comprenant un tronçon s'étendant radialement et un segment médian en forme de U, la profondeur du segment en forme de U étant différente pour chaque fil métallique d'ossature (11), de telle sorte que les tronçons, s'étendant radialement, des fils métalliques d'ossature (11) forment un plan, le plan étant recouvert par une membrane,
**caractérisé en ce que**
le disque droit (21) est réalisé sous forme d'un treillis métallique et les fils métalliques d'ossature (11) du disque gauche (1) forment le plan après avoir été reliés conjointement l'un à l'autre, et
les fils métalliques d'ossature (11) passent à travers le treillis métallique du disque droit (21) pour relier le disque droit (21) au disque gauche (1), de telle sorte que les disques (1, 21) du dispositif d'occlusion puissent osciller à volonté, afin d'être parallèles ou d'avoir un angle l'un par rapport à l'autre.

2. Dispositif selon la revendication 1, dans lequel le disque gauche (1) est formé par plusieurs fils métalliques d'ossature (11) s'étendant radialement et est couvert par des membranes (100) sur les deux côtés du plan pour stopper le flux de sang.

3. Dispositif selon la revendication 2, dans lequel les deux extrémités des fils métalliques d'ossature (11) ont une forme sphérique et les deux sphères sont enveloppées dans une membrane (100) sur les deux côtés du plan.

4. Dispositif selon l'une des revendications 1 à 3, dans lequel le disque droit (21) est formé par un treillis métallique tubulaire qui subit un traitement thermique pour former une structure de disque et qui est fixé à une extrémité par une pointe (22) et à l'autre par une articulation (23), respectivement.

5. Dispositif selon la revendication 4, dans lequel les fils métalliques d'ossature (11) passent à travers le treillis métallique du disque droit (21) à proximité de la pointe (22).

6. Dispositif selon la revendication 5, dans lequel vus en direction axiale du disque gauche (1), les fils métalliques d'ossature (11) se chevauchent lorsqu'ils passent à travers le treillis métallique à proximité de la pointe (22) du disque droit (21).

7. Dispositif selon l'une des revendications 1 à 6, dans lequel les membranes (100) sont réalisées en matériaux biocompatibles.

8. Dispositif selon la revendication 1, dans lequel les fils métalliques d'ossature (11) sont réalisés en fil de nitinol à mémoire de forme.

9. Dispositif selon la revendication 1, dans lequel les disques gauche et droit (1, 21) tendent à se contracter vers l'intérieur en direction l'un de l'autre et peuvent être orientés parallèlement l'un à l'autre ou présenter un angle afin de fournir la fonction d'auto-ajustement pour des défectuosités de différentes épaisseurs et pour permettre une fixation proche de la défectuosité.
